# EUROPEAN PATENT APPLICATION

(11) **EP 1 935 872 A1**
(43) Date of publication of application: **25.06.2008**
(21) Application number: 06125842.2
(22) Date of filing: 11.12.2006
(51) Int. Cl.: C07C 213/10, C07C 215/60

(54) **Process for obtaining the R-enantiomer of salbutamol**

(71) Applicant: Stirling Products Limited, Perth WA 6000 (AU)
(72) Inventor: Stolz, Florian, 79211 Denzlingen (DE); Balint, Joszef, 12524 Berlin (DE)
(74) Representative: Reitstötter - Kinzebach

(57) **Abstract**

The present invention relates to a process for obtaining the R-enantiomer of salbutamol from a mixture of the enantiomers of salbutamol by a crystallization of said R-enantiomer as its salt with R-ibuprofen from a solution or slurry of a mixture of the enantiomers of salbutamol.

## Description

The present invention relates to a process for obtaining the R-enantiomer of salbutamol from a mixture of the enantiomers of salbutamol by a crystallization of said R-enantiomer as its salt with a chiral acid from a solution or slurry of a mixture of the enantiomers of salbutamol.

### BACKGROUND OF THE INVENTION

Salbutamol (also referred to as albuterol or α-[[(1,1-dimethylethyl)amino]methyl]-4-hydroxy-1,3-benzenedimethanol) is a β₂-agonist useful as a bronchodilator and is thus used in the treatment of respiratory diseases such as asthma and related medical conditions. It is known that the (R)-isomer of salbutamol (which is laevorotatory) is about 80 times more active than the dextrorotatory (S)-isomer, and research has indicated that administration of the pure (R)-enantiomer may offer an improved therapeutic ratio. Recently, the use of the (R)-enantiomer of salbutamol as a feed additive has been suggested (US6110974). Therefore, much effort has been spent to provide the pure (R)-enantiomer of salbutamol.

EP 763010 discloses a process for obtaining single enantiomers of salbutamol including the optical resolution of methyl 5-[2-[(1,1-dimethylethyl)amino]-1-hydroxyethyl]-2-(phenylmethoxy)benzoat or α-[[(1,1-dimethylethyl)amino]methyl]-4-(phenylmethoxy)-1,3-benzenedimethanol by means of a chiral tartaric acid derivative such as (-)-di-toluoyl-L-tartaric acid and (+)-di-toluyl-D-tartaric acid in methanol.

US 6,365,756 describes the resolution of a new ketal derivative of salbutamol (specifically 2-(N-t-butylamino)-1-(2,2-dimethyl-1 ,2-benzodioxin-6-yl)ethanol). The resolution is again performed using a chiral tartaric acid derivative.

The chiral tartaric acid derivatives used, however, are expensive and, therefore, the use of these compounds renders these processes unattractive from an economical point of view. Moreover, the reported enantiomeric excess is not satisfactory. Furthermore, only synthetic precursors or derivatives of salbutamol can be used and not salbutamol itself. Therefore, these methods require additional synthesis steps or the synthesis is limited to a single synthetic pathway.

WO 2002/048090 suggests the use of (L)-tartaric acid for resolving a racemic mixture of salbutamol or salbutamol precursors. However, the overall yield for optical resolution of salbutamol is not satisfactory.

R.P. Bakale et al., Clinical Reviews in Allergy and Immunology 14, 1996, pp. 7 to 35 describe the optical resolution of racemic salbutamol by crystallizing R-salbutamol as its acid addition salt with S-Naproxen. However the yield is rather low (15 % yield; 86% ee). Bakale et al. also report about a screening of a large number of chiral acids, including R-ketoprofen, S-ibuprofen, L-tartaric acid, (+)-DBTA, (-)-DTTA, S-malic acid, L-aspartic acid, L-glutamic acid, mandelic acid, camphor sulfonic acid and R-chloromethylphenoxypropionic acids with regard to their capability for optical resolution of RS-salbutamol. However, no enrichment was reported for these acids.

Argentine Patent Application No. 990102613 discloses a process for the optical resolution of a racemic mixture of salbutamol sulfate. The process includes the enrichment of racemic salbutamol sulfate with the desired enantiomer of salbutamol sulfate, forming a supersaturated solution of the thus enriched salbutamol sulfate and inducing crystallization by seeding with small amounts of the desired enantiomer of salbutamol sulfate. The process is not satisfactory for several reasons. First, large amounts of solvent are required, whereby the space-time-yield of the crystallization is lowered. Second, the reported enantiomeric enrichment is not satisfactory despite the prior enrichment with the desired enantiomer.

Earlier European patent application 06004756.0 discloses a process for the enantiomeric enrichment of salbutamol and salbutamol precursors and the acid-addition salts thereof, the process comprising the crystallization of salbutalmol or salbutamol-precursor as its acid-addition salt with an achiral carboxylic acid A that has at least three carbon atoms and a solubility of less than 50 g/l in water at pH < 3, 20°C and 1013 mbar, from a solution containing a mixture of the enantiomers of the compound of salbutamol or salbutamol precursor and the achiral carboxylic acid A in the presence of seed crystals of the desired enantiomer or the acid addition salt thereof, whereby the enantiomerically enriched acid-addition salt of salbutamol or salbutamol precursor is obtained.

Nevertheless, there is still a strong need for providing a process for obtaining enantiomerically enriched or pure R-salbutamol.

### SUMMARY OF THE INVENTION

It was now surprisingly found that optical resolution of a mixture of salbutamol enantiomers can be achieved by subjecting a mixture of salbutamol enantiomers to a crystallization in the presence of R-ibuprofen (R-enantiomer of 2-(4-isobutylphenyl)-propionic acid). Thereby, a selective crystallization of the acid addition salt of R-salbutamol with R-ibuprofen is achieved.

Thus, in a first aspect the present invention provides a process for obtaining the R-enantiomer of salbutamol (hereinafter also referred to as R-salbutamol) or a pharmaceutically acceptable salt thereof, which comprises crystallizing said R-enantiomer as its salt with a chiral acid from a solution or slurry of a mixture of the enantiomers salbutamol in at least one solvent, wherein the chiral acid is R-ibuprofen.

Furthermore, it was surprisingly found that optical resolution of a mixture of salbutamol enantiomers can also be achieved by subjecting a mixture of salbutamol enantiomers to a crystallization in the presence of S-ibuprofen (S-enantiomer of 2-(4-isobutylphenyl)propionic acid). Thereby, a selective crystallization of the acid addition salt of the S-enantiomer of salbutamol (S-salbutamol) with S-ibuprofen is achieved. The thus obtained mother liquor is highly enriched with R-salbutamol, which can be selectively crystallized from said mother liquor by standard crystallization techniques.

Thus, in a second aspect the present invention provides a process for obtaining the R-enantiomer of salbutamol or a pharmaceutically acceptable salt thereof, which comprises
a) crystallizing S-salbutamol as its salt with a chiral acid from a solution or slurry of a mixture of said R- and S-enantiomers in at least one solvent, wherein the chiral acid is S-ibuprofen, and
b) crystallizing R-salbutamol or a salt thereof from the mother liquor obtained in step a).

The thus obtained acid addition salts of R-salbutamol with R-ibuprofen or S-salbutamol with S-ibuprofen are of high optical purity, the enantiomeric excess (ee.) being generally at least 70 %, frequently at least 80 % preferably at least 86 %, in particular at least 90 %, corresponding to a R:S-ratio (weight ratio of R-salbutamol to S-salbutamol) or S:R-ratio, respectively, of at least 85:15, frequently at least 90:10, preferably at least 93:7 and in particular at least 95:5, respectively. Moreover, the optical purity can be further improved by simple recrystallization. The optical purity, i.e. the enantiomeric excess with regard to salbutamol is then generally higher than 90 %, in particular at least 95 %, and more preferably at least 98 % or 99 %.

The acid addition salt of R-salbutamol with R-ibuprofen (hereinafter also referred to as R-salbutamol*R-ibuprofen) is new and is also part of the present invention. The optical purity of the salt in terms of enantiomeric excess (with regard to the desired R-Salbutamol) is generally at least 70 %, frequently at least 80%, preferably at least 90 %, in particular at least 95 %, and more preferably at least 98 % or 99 %.

The acid addition salt of S-salbutamol with S-ibuprofen (hereinafter also referred to as S-salbutamol*S-ibuprofen) is new and is also part of the present invention. The optical purity of the salt in terms of enantiomeric excess (with regard to S-Salbutamol) is generally at least 70 %, frequently at least 80%, preferably at least 90 %, in particular at least 95 %, and more preferably at least 98 % or 99 %.

### DETAILED DESCRIPTION OF THE INVENTION

According to a first aspect of the present invention, the acid addition salt of R-salbutamol with R-ibuprofen is crystallized from a slurry or preferably a solution containing the mixture of salbutamol enantiomers suspended or dissolved, respectively, in a suitable solvent or solvent mixture.

The mixture of the salbutamol enantiomers contained in the slurry or the solution, may be a racemic mixture or a non-racemic mixture. If a non-racemic mixture is used, the excess of one enantiomer will generally not exceed 30 %, in particular 20 %, i. e. the R:S-ratio will be in the range from 65:35 to 35:65, in particular in the range from 60:40 to 40:60. Such non-racemic mixtures may be obtained during crystallization of one of the enantiomers from a solution of the racemic mixture of salbutamol or by addition of racemic salbutamol to a mother liquor from which one of the enantiomers had already been crystallized. Racemic mixtures as well as non-racemic mixtures can be used both in the process according to the first aspect of the present invention as well as in the process according to the second aspect of the present invention.

The crystallization of R-salbutamol*R-ibuprofen by the process according to the first aspect of the present invention can be achieved by analogy to standard techniques for the optical resolution of enantiomeric mixtures of compounds having a basic nitrogen with chiral acids, e.g. by analogy the methods described in the prior art cited in the introductory part of the present application.

In particular, the crystallization of R-salbutamol is performed in the presence of suitable amounts of R-ibuprofen. Needless to say, the amount of R-ibuprofen is chosen to ensure crystallization of the acid addition salt R-salbutamol*R-ibuprofen. Usually, the amount of R-ibuprofen used is at least 0.8 mol, in particular at least 0.9 mol per mol of R-salbutamol which is present in the solution or the slurry before crystallization. Preferably, R-ibuprofen is used in an amount of 0.9 to 3.0 moles per mol of R-salbutamol which is contained in the solution or slurry before crystallization. Preferably, R-ibuprofen is used in an amount of 0.9 to 2.2 moles per mol of R-salbutamol which is contained in the solution or slurry before crystallization, when the solution or slurry contains a racemic mixture or a mixture of salbutamol enantiomers, wherein the relative amount of R- and S-enantiomer is close to 1:1 (i.e. from 45:55 to 55:45).

In one embodiment of the process according to the first aspect of the present invention, R-ibuprofen is used in a molar amount which is close to the total molar amount of salbutamol which is present in the solution or slurry, i. e. the molar ratio of R-ibuprofen to the mixture of salbutamol enantiomers is from 0.8:1.2 to 1.2:0.8, in particular from 0.9:1.1 to 1.1:0.9. In particular, the molar ratio of R-ibuprofen to the total amount of salbutamol is at least 1:1, or from 1:1 to 1.1:1.

In a second embodiment of the process according to the first aspect of the invention, R-ibuprofen is used together with an achiral acid. In this embodiment, the total amount of achiral acid plus R-ibuprofen is preferably close to the required amount for complete neutralization of the salbutamol present in the mixture before crystallization. In other words, the total amount of achiral acid plus R-ibuprofen is from 0.8 to 1.2 mol, in particular from 0.9 to 1.1 mol per mol of salbutamol present in the solution or slurry before crystallization. In this embodiment, R-ibuprofen is used in an amount of at least 0.8 mol, in particular at least 0.9 mol, preferably from 0.8 to 1.4 mol, in particular from 0.9 to 1.2 mol per mol of R-salbutamol present in the slurry or solution before crystallization.

Suitable achiral acids which can be used in combination with R-ibuprofen may be organic acids or inorganic acids, in particular those acids which do not decrease the solubility of salbutamol in the solvent which forms the solution or slurry. Suitable acids include in particular hydrochloric acid, formic acid or acetic acid.

For the crystallization process according to the first aspect of the invention, the mixture of the salbutamol enantiomers is dissolved or suspended in a suitable solvent or solvent mixture. Preferably, the mixture of salbutamol enantiomers is completely dissolved prior to crystallization.

The solution of the salbutamol enantiomers can be a homogeneous solution, i. e. the mixture of enantiomers and at least one solvent form a single phase prior to crystallization, or a heterogeneous solution (multi-phase solution), wherein the mixture of enantiomers is dissolved in at least one solvent, preferably in a mixture of at least two solvents of different polarity, thereby forming a multi-phase liquid. Both, the homogeneous solution and the emulsion may further comprise solid material, in particular undissolved salbutamol enantiomers or undissolved chiral acid. Preferably, no undissolved salbutamol is present prior to crystallization.

Suitable solvents include in particular water, organic solvents that have miscibility with water of at least 20 % at room temperature, mixtures thereof with water as well as mixtures of water or said water miscible solvents with organic solvents that have miscibility with water of less than 20 % at room temperature.

Suitable solvents having a water miscibility of at least 20 % at room temperature include, but are not limited to:
1. C₁-C₄-Alkanols such as methanol, ethanol, n-propanol or isopropanol;
2. Amides and N,N-dimethylamides of C₁-C₃-carboxylic acids such as formamide, dimethylformamide (DMF), acetamide and N,N-dimethylacetamide;
3. 5 or 6-membered lactames with a total of 7 carbon atoms such as pyrrolidone, N-methylpyrrolidone, N-ethylpyrrolidone, N-isopropylpyrrolidone, N-hydroxyethylpyrrolidone;
4. Amides, methyl- and dimethylamides of C₁-C₃-carboxylic acids such as formamide, dimethylformamide and dimethylacetamide,
5. Dimethylsulfoxid and sulfolane;
6. Ketones with 3 to 6 carbon atoms such as acetone, 2-butanone, cyclopentanone and cyclohexanone;
7. Acetonitrile;
8. 5- or 6-membered lactones such as γ-butyrolactone;
9. Polyols and polyetherols such as glycol, glycerin, dimethoxyethan, ethylendiglycol, ethylenglycolmonomethylether, etc, and
10. Cyclic ethers such as tetrahydrofurane, dioxane and trioxane, dimethyl (poly)C₂-C₃-alkyleneglycol ethers such as dimethoxyethane, diethyleneglycoldimethylether, triethyleneglycoldimethylether, dipropyleneglycoldimethylether, low molecular weight polyethyleneglycoles and low molecular weight polypropyleneglycoles (MW ≤ 400).

Preferred solvents for the crystallization process according to the first aspect of the present invention comprise at least one protic solvent, which is selected from water and C₁-C₄-alkanols such as methanol, ethanol, n-propanol or isopropanol. These solvents may be used as such or in a mixture with an aprotic solvent, in particular an aprotic solvent having a reduced water solubility, e.g. an aprotic solvent having a water solubility of ≤ 20 % v/v, especially ≤ 5 % v/v at room temperature.

Aprotic solvents having a water solubility of ≤ 20 % v/v, in particular ≤ 5 % v/v at room temperature include, but are not limited to:
1. aromatic solvents, such as benzene or its derivatives like toluene, benzonitrile, nitrobenzene, chlorobenzene or xylene and heteroaromatic liquids such as pyridine or furane;
2. halogenated alkanes like dichloromethane, dichloroethane, trichloroethane;
3. alkyl ethers having ≥ 4, e.g. 4 to 10 carbon atoms, such as diethylether, diisopropyl ether or tert-butylmethylether;
4. esters of n-, i- or branched carboxylic acids with ≥ 5 carbon atoms, including diesters, triesters, such as oils and fats, and polyesters;
5. alkanoles, aromatic and cyclic alcohols with ≥ 5, e.g. 5 to 10 carbon atoms, e.g. 2-hexanol, cyclohexanol, benzylalcohol or octanol.

In a very preferred embodiment of the process according to the first aspect of the present invention, the solvent which is used for forming the solution or slurry is a mixture of water with at least one organic solvent, which is selected from C₁-C₄-alkanols and aprotic solvents. In particular, said mixture comprises, besides water, at least one of the aforementioned solvents having a reduced water solubility. In particular, the solvent which is used in the process according to the first aspect of the present invention is a mixture of water with at least one aprotic solvent having a water miscibility of ≤ 20 % v/v at room temperature, more preferably a mixture of water with a suitable dialkyl ether having from 4 to 10 carbon atoms, such as tert.-butyl methylether, tert.-butyl ethylether, diisopropylether, diethylether, and the like.

In another preferred embodiment of the process according to the first aspect of the present invention the solvent is C₁-C₄-alkanol such as methanol, ethanol, n-propanol or isopropanol, a mixture thereof or a mixture thereof with water, with preference giving to methanol, ethanol and mixtures thereof with water, wherein the amount of water is less than 30 % v/v

The crystallization of the acid addition salt R-salbutamol*R-ibuprofen from the solution or slurry containing the mixture of salbutamol enantiomers may be achieved by conventional crystallization techniques know in the art. Usually, a mixture of the salbutamol enantiomers is suspended or preferably dissolved in a suitable solvent to obtain a slurry or preferably a solution of the salbutamol in said solvent.

The concentration of salbutamol in the slurry or solution is usually from 5 to 55 % by weight, in particular from 10 to 45 % by weight and more preferably from 15 to 40 % by weight, based on the total amount of salbutamol and solvent.

To the solution R-ibuprofen and optionally the achiral acid is added in order to effect crystallization of the acid addition salt R-salbutamol*R-ibuprofen. R-Ibuprofen may be added as a solid, e.g. as a powder or crystalline material, or as a solution. R-Ibuprofen may be added at once or in portions or continuously.

The temperature of the solution or slurry to which R-ibuprofen is added is of minor importance and may vary from 0°C to 100°C, in particular from 15 to 60°C and is more preferably at about room temperature, i.e. from 20 to 30°C. R-Ibuprofen may be added to a hot solution (temperature > 35°C) or the slurry or solution may be warmed to a temperature above 35°C after the addition of ibuprofen. However, it is not necessary to warm the solution or slurry before or after the addition of R-ibuprofen.

If R-ibuprofen is added to a solution having a temperature of at least 35°C or the mixture is warmed to a temperature of at least 35°C, the crystallization is effected by lowering the temperature. Usually the temperature is lowerd by at least 5 K, in particular at least 10 K, e.g. by 10 to 30 K. The cooling rate will usually be in the range from 1 to 10 K/h.

In a very preferred embodiment of the process according to the first aspect of the invention, R-ibuprofen is added as a solid, in particular as crystals at a temperature which does not exceed 40°C, in particular at a temperature from 20 to 30°C and the crystallization is performed in the same temperature range.

The thus formed acid addition salt R-salbutamol*R-ibuprofen is then separated from the mother liquor by conventional separation techniques such as filtration and centrifugation. The thus obtained crystalline material may be washed with a suitable solvent, e. g. with water or a solvent in which the acid addition salt is only sparingly soluble or even insoluble to remove mother liquor and further impurities, in particular acid addition salts of S-salbutamol.

In the acid addition salt R-salbutamol*R-ibuprofen obtained by the process according to the first aspect of the present invention the molar ratio of R-salbutamol to S-salbutamol (hereinafter also referred to as R:S-salbutamol) is usually at least 90:10, in particular at least 93:7. However, optical purity can be further improved by recrystallization of said acid addition salt from a suitable solvent. Suitable solvents include but are not limited to C₁-C₄-alkanols, in particular methanol or ethanol, and mixtures thereof with water.

The thus obtained acid addition salt R-salbutamol*R-ibuprofen can be transformed into salbutamol or pharmaceutically acceptable acid addition salts of salbutamol according to well-known techniques, e.g. as described in the prior art cited in the introductory part of the present application. For the preparation of a pharmaceutically acceptable acid-addition salt, such as the sulfate, hydrogen sulfate or hydrochloride, R-salbutamol*R-ibuprofen is usually transformed into the free base and then treated with an acid that provides the pharmaceutical acceptable counter ion. The transformation of R-salbutamol*R-ibuprofen into the free base is usually achieved by treatment with a diluted aqueous base, in particular an aqueous solution of an alkalimetal carbonate, alkalimetal hydrogen carbonate or alkalimetal hydroxide such as sodium carbonate, potassium carbonate, sodium hydrogencarbonate, sodium hydroxide, calcium hydroxide or potassium hydroxide, optionally in the presence of an organic solvent to assist solubilization of the free base. Optionally, the carboxylic acid is removed with a basic ion exchange resin. The thus obtained salbutamol free base is then treated with a solution of the desired acid in water or in a suitable organic solvent. It is, however, also possible to transform the acid-addition R-salbutamol*R-ibuprofen into the pharmaceutically acceptable acid-addition salt by direct treatment of the acid-addition salt with a strong acid that provides the pharmaceutically acceptable counter ion. In this context, the term "strong acid" has to be understood as an acid having a pKs below the pKs of ibuprofen. For example, R-salbutamol*R-ibuprofen obtained from the crystallization is dissolved in a suitable solvent, e. g. in a C₁-C₄-alkanol and the strong acid, e.g. sulfuric acid or a sulfonic acid, providing the pharmaceutically acceptable counter ion is added to precipitate the pharmaceutically acceptable acid-addition salt. The solution obtained by either the direct or indirect transformation of R-salbutamol*R-ibuprofen into its pharmaceutically acceptable acid addition salt contains R-ibuprofen which is of commercial interest and which can be recovered from the solution by techniques known in the art, e.g. simply by precipitating R-ibuprofen from the mother liquor by diluting the solution with water.

After having separated R-salbutamol*R-ibuprofen the obtained mother liquor still contains some R-salbutamol together with an excess of S-salbutamol. The contained S-Salbutamol may be depleted from the obtained mother liquor and the thus obtained mother liquor may be re-loaded with a racemic or non-racemic mixture of the salbutamol enantiomers and the crystallization of R-salbutamol*R-ibuprofen can be again performed as outlined above. The crystallization of S-salbutamol from the mother liquor can be achieved by any techniques effecting the crystallization of salbutamol or of a suitable acid addition salt, e. g. by concentrating the mother liquor, by cooling the mother liquor or by adding an acid which reduces the solubility of S-salbutamol in the mother liquor or by any combination of these measures. Preferably, S-salbutamol is crystallized as an acid addition salt which has a reduced solubility in the mother liquor. In particular, S-salbutamol is crystallized from the mother liquor as an acid addition salt with a suitable achiral acid, more preferably as an acid addition salt with an achiral acid that provides reduced solubility. Suitable acids which lead to a reduced solubility of S-salbutamol in the mother liquor include sulfuric acid, hydrochloric acid or pivalic acid (or S-Ibuprofen). In a very preferred embodiment, S-salbutamol is crystallized from the mother liquor as its sulfate (S-salbutamol*0.5 H₂SO₄).

The non-desired S-salbutamol can be racemized by standard techniques, e. g. by treatment with a strong acid such a sulfuric acid, hydrochloric acid or toluene sulfonic acid at elevated temperature. In particular, racemisation can be performed by analogy to example 15 described in US 6,365,756. The thus obtained racemized mixture of salbutamol enantiomers can be subjected to a crystallization according to the present invention, thereby improving the overall yield of the desired enantiomer.

In the process according to the second aspect of the invention S-salbutamol is crystallized from a solution or slurry containing a mixture of the salbutamol enantiomers as described above. In particular, the mixture of enantiomers is a racemic mixture. However, the non-racemic mixtures described above can likewise be employed.

By the crystallization of the S-salbutamol as its acid addition salt with S-ibuprofen, the mother liquor is depleted with regard to S-salbutamol or enriched with regard to R-salbutamol, respectively. Consequently, the mother liquor can be used as a source for R-salbutamol, from which R-salbutamol may be crystallized as such or as an acid addition salt, in particular as its sulfate, hydrogen sulfate, hydrochloride, pivalic acid salt or R-ibubrofen salt (step b), more preferably as its sulfate.

In the first step a) of the process according to the second aspect of the present invention, S-salbutamol*S-ibuprofen is crystallized from a slurry or a solution that contains the mixture of the salbutamol enantiomers. Step a) of the process according to the second aspect of the invention can be performed similar to the crystallization of R-salbutamol*R-ibuprofen as described above for the process according to the first aspect of the present invention, to which full reference is made.

In a preferred embodiment of step a), S-ibuprofen is used in a molar amount which is close to the total molar amount of salbutamol that is present in the solution or slurry, i. e. the molar ratio of S-ibuprofen to the mixture of salbutamol enantiomers is from 0.8:1.2 to 1.2:0.8, in particular from 0.9:1.1 to 1.1:0.9. In particular, the molar ratio of S-ibuprofen to the total amount of salbutamol is at least 1:1, more preferably from 1:1 to 1.1:1.

Preferred solvents for step a) comprise at least one protic solvent, which is selected from water and C₁-C₄-alkanols such as methanol, ethanol, n-propanol or isopropanol. These solvents may be used as such or in a mixture with an aprotic solvent, in particular an aprotic solvent having a reduced water solubility, e.g. an aprotic solvent having a water solubility of ≤ 20 % v/v, especially ≤ 5 % v/v at room temperature. In a very preferred embodiment of step a) the solvent is a C₁-C₄-alkanol such as methanol, ethanol, n-propanol or isopropanol, a mixture thereof or a mixture thereof with water, with preference given to C₁-C₄-alkanols, in particular methanol or ethanol, and mixtures thereof with water, wherein the amount of water is less than 30 % v/v.

The temperature of the solution or slurry to which S-ibuprofen is added is of minor importance and may vary from 10 to 100°C, in particular from 15 to 60°C and is more preferably about room temperature, i.e. from 20 to 30°C. S-Ibuprofen may be added as a solid or as a solution. S-Ibuprofen may be added to a hot solution (temperature ≥ 35°C) or the slurry or solution may be warmed to a temperature above 35°C after the addition of ibuprofen. However, it is not necessary to warm the solution or slurry before or after the addition of R-ibuprofen. If S-ibuprofen is added to a solution having a temperature of at least 35°C or the mixture is warmed to a temperature of at least 35°C, the crystallization is effected by lowering the temperature. Usually the temperature is lowered by at least 5 K, in particular at least 10 K, e.g. by 10 to 30 K. The cooling rate will usually be in the range from 1 to 10 K/h.

The acid addition salt S-salbutamol*S-ibuprofen which is formed during crystallization is then separated from the mother liquor by conventional separation techniques such as filtration and centrifugation. The thus obtained crystalline material may be washed with a suitable solvent, e. g. with water or a solvent in which the acid addition salt is only sparingly soluble or even insoluble to remove further impurities, in particular acid addition salts of R-salbutamol.

In the acid addition salt S-salbutamol*S-ibuprofen obtained in step a) the molar ratio of R-salbutamol to S-salbutamol (hereinafter also referred to as R:S-salbutamol) is usually at least 9:1, in particular at least 93:7. However, optical purity can be further improved by recrystallization of said acid addition salt from a suitable solvent as outlined above for R-salbutamol*R-ibuprofen

In step b) of the process according to the second aspect of the present invention, the R-salbutamol is crystallized from the mother liquor obtained in step a).

The crystallization can be achieved by any techniques effecting the crystallization of salbutamol or of a suitable acid addition salt, e. g. by concentrating the mother liquor, by cooling the mother liquor or by adding an acid which reduces the solubility of R-salbutamol in the mother liquor or by any combination of these measures.

In a preferred embodiment of step b) R-salbutamol is crystallized as an acid addition salt which has a reduced solubility in the mother liquor. In particular, R-salbutamol is crystallized from the mother liquor as an acid addition salt with a suitable achiral acid, in particular as an acid addition salt with an achiral acid that provides a pharmaceutically acceptable counter-ion. Suitable acids which lead to a reduced solubility of R-salbutamol in the mother liquor include sulfuric acid, hydrochloric acid, pivalic acid. Likewise, R-ibuprofen can be used. In a very preferred embodiment, R-salbutamol is crystallized from the mother liquor as its sulfate (R-salbutamol*0.5 H₂SO₄).

If R-salbutamol is crystallized from the mother liquor as a suitable acid addition salt, step b) is usually performed by adding a suitable acid to the mother liquor obtained in step a). The mother liquor may be warmed prior to the addition of said acid or after the addition. However, a warming of the mother liquor is not necessary and usually the acid is added at a temperature in the range from 0°C to 40°C, in particular from 20 to 30°C and the crystallization is performed at the same temperature range. However, it is also possible to cool the mixture during crystallization to increase yield.

It may be suitable to replace some of the solvent present in the mother liquor obtained in step a) with another solvent that reduces the solubility of R-salbutamol or of its acid addition salt in the mother liquor. However, such a solvent replacement is in principle not necessary.

The thus obtained R-salbutamol or its acid addition salt can be separated from the mother liquor by suitable techniques of separating solid materials from liquids, including centrifugation and filtration. In order to remove mother liquor and further impurities, the thus obtained crystalline material can be washed with a suitable solvent or solvent mixture, wherein R-salbutamol or its acid addition salt is sparingly soluble or even insoluble, suitable solvents including water, C₁-C₄-alkanols, such as methanol and mixtures thereof with water.

In step b), a further mother liquor is obtained which has been depleted with regard to both R-salbutamol and S-salbutamol but which usually still contains some salbutamol and ibuprofen dissolved therein. Therefore, this mother liquor may be loaded again with a mixture of the salbutamol enantiomers, in particular with racemic salbutamol and steps a) and b) may be repeated. By this measure, loss of salbutamol and ibuprofen can be further reduced and overall yield can be increased.

The S-salbutamol contained in the acid addition salt S-salbutamol*S-ibuprofen obtained by the process according to the second aspect of the present invention may be discarded or may be subjected to a racemisation as described above.

The following examples shall serve the further illustration of the invention and are not intended to limit the scope of the present invention.

### Example 1: Resolution of racemic salbutamol with R-ibuprofen

A reaction vessel, equipped with a stirrer, was charged with a mixture of 43.8 kg of racemic salbutamol, 72 kg of water and 28 kg of tert.-butylmethylether. To this mixture 8.8 kg of concentrated hydrochlorid acid (37%) and 19.8 kg of R-ibuprofen were added. The mixture was stirred for 12 h at 23°C during which time crystalline material formed. The crystalline material was separated from the mother liquor by centrifugation and the obtained crystals were washed three times each with 10 kg of water and then dried for 18 h at 40°C. Thereby, 34.9 kg (87% of theory) of the acid addition salt of R-salbutamol with R-ibuprofen were obtained. In the obtained material, the ratio of R-salbutamol to S-salbutamol (R:S-salbutamol) was 93:7 (HPLC; column: chirobioticV (Astec); eluent: methanol/acetic acid/triethylamine 100:0.02:0.02)

### Example 2: Recrystallization of R-salbutamol*R-ibuprofen

33 kg of R-salbutamol*R-ibuprofen having an R:S-salbutamol ratio of 93:7 were suspended in 28 kg of methanol. The mixture was stirred at 40°C for one hour. Then the mixture was cooled to 22°C with stirring over a period of 2 h and stirred at 22°C for further 50 minutes. The obtained crystalline material was separated by centrifugation, washed with 5 kg acetone and tried to yield 25.6 kg (78 % of theory) of R-salbutamol*R-ibuprofen having an enantiomeric ratio R:S-salbutamol of > 99:1.

Repeated re-crystallization from methanol as described above yields R-salbutamol *R-ibuprofen having an enantiomeric ratio R:S-Salbutamol > 99.5%. (HPLC, see above)

### Example 3: Preparation of R-salbutamol sulfate

21 kg of the acid addition salt of R-salbutamol with R-ibuprofen having an enantiomeric ratio R:S-salbutamol of 99.5:0.5 were added to a mixture of 32.5 kg of methanol and 2.55 kg of concentrated sulfuric acid (96%). The mixture was stirred at room temperature for two hours. Then the crystalline material obtained was separated by centrifugation, washed with methanol and dried to yield 12.5 kg (92 %) of R-salbutamol sulfate (salbutamol *0.5 H₂SO₄). Chemical purity: 99.5% (HPLC, see above)

To the thus obtained mother liquor 50 kg of water were added. After stirring the mixture for 18 h the formed crystalline material was filtered of, washed with water and dried to obtain 8.8 kg (91 %) of R-ibuprofen. The thus obtained R-ibuprofen has a chemical purity of >98% (HPLC) and could be used in a next crystallization cycle.

### Example 4: Resolution of racemic salbutamol with R-ibuprofen by crystallization from ethanol

To a solution of 2.39 g of racemic salbutamol in 6 g of ethanol (96 %) 2.6 g of R-ibuprofen were added and the mixture was warmed to 50°C. After stirring at 50°C for ten minutes, the mixture was cooled to 25°C and stirred for further four hours. Then, the obtained crystalline material was separated by filtration, washed with ethanol and dried to yield 1.6 g (73 % of theory) of the acid addition salt of R-salbutamol with R-ibuprofen. The obtained material had an enantiomeric ratio R:S-salbutamol of 99:1.

### Example 5: Optical resolution of racemic salbutamol with S-ibuprofen, including subsequent crystallization of R-salbutamol *0.5 H₂SO₄

Step A: To a solution of 23.9 g of racemic salbutamol in 40 g methanol 20.6 g of S-ibuprofen were added and the mixture was warmed to 40°C. After stirring at 40°C for 10 min., the solution was cooled to 25°C and seeded with 10 mg of the acid addition salt of S-salbutamol with S-ibuprofen. The mixture was stirred for further 18 hours at 25°C. Then, the formed crystalline material was separated by filtration, washed with methanol and dried to yield 17 g of the acid addition salt of S-salbutamol with S-ibuprofen having an enantiomeric ratio of 91:9 (S:R).

Step B: To the mother liquor of step A, 1.05 g of concentrated sulfuric acid (96 %) were added and the mixture was stirred for further two hours at 23°C. The formed crystalline material was separated from the mother liquor by filtration, washed with methanol and dried to yield 6.2 g of R-salbutamol sulfate having an enantiomeric ratio of 91:9 (R:S).

Step C: To the mother liquor of step B 17.6 g of racemic salbutamol and 12.3 g of S-ibuprofen were added. The enantiomeric ratio R:S was about 54:46. The mixture was heated to 40°C and stirred for ten minutes at 40°C. Then the mixture was cooled to 25°C, seeded with 10 mg of S-salbutamol*S-ibuprofen and stirred for further 18 hours. The thus formed crystalline material was separated from the mother liquor by filtration, washed with methanol and dried to yield 16.3 g of the acid addition salt of S-salbutamol with S-ibuprofen having an enantiomeric ratio of 87:13 (S:R).

Step D: To the mother liquor of step C 1.05 g of concentrated sulfuric acid (96 %) were added and the mixture was stirred for two hours at 23°C. The thus formed crystalline material was separated from the mother liquor by filtration, washed with methanol and dried to yield 6.7 g of R-salbutamol *0.5 H₂SO₄ having an enantiomeric ratio of 93:7 (R:S).

## Claims

1. A process for obtaining the R-enantiomer of α-[[(1,1-dimethylethyl)-amino]methyl]-4-hydroxy-1,3-benzenedimethanol or a pharmaceutically acceptable salt thereof, which comprises crystallizing said R-enantiomer as its salt with a chiral acid from a solution or slurry of a mixture of the enantiomers of α-[[(1,1-dimethylethyl)amino]methyl]-4-hydroxy-1,3-benzenedimethanol in at least one solvent, wherein the chiral acid is R-Ibuprofen.

2. The process according to claim 1, wherein the crystallization is effected from a solution or slurry containing a mixture of the enantiomers of α-[[(1,1-dimethylethyl)amino]methyl]-4-hydroxy-1 ,3-benzenedimethanol wherein the at least one solvent forming the solution or slurry comprises at least one protic solvent, selected from water and C₁-C₄-alkanols.

3. The process according to claim 2, wherein the at least one solvent is a mixture of water with an organic solvent, selected from C₁-C₄-alkanols and aprotic organic solvents.

4. The process according to any of the preceding claims, wherein the chiral acid is used in an amount of 0.9 to 3.0 moles per mol of the R-enantiomer of α-[[(1,1-dimethylethyl)amino]methyl]-4-hydroxy-1,3-benzenedimethanol which is contained in the solution or slurry before crystallization.

5. The process according to any of the preceding claims, wherein the solution or slurry before crystallization additionally contains at least one achiral acid, the total amount of achiral acid and chiral acid being at least 0.9 equivalent per mol of α-[[(1,1-dimethylethyl)amino]methyl]-4-hydroxy-1,3-benzenedimethanol which is contained in the solution or slurry before crystallization.

6. The process according to any of the preceding claims, wherein the mother liquor obtained from the crystallization is subjected to a racemisation of the α-[[(1,1-dimethylethyl)amino]methyl]-4-hydroxy-1,3-benzenedimethanol contained in the mother liquor.

7. The process according to any of the preceding claims, further comprising the transformation of the obtained salt of (R)-α-[[(1,1-dimethylethyl)amino]methyl]-4-hydroxy-1,3-benzenedimethanol with R-Ibuprofen into a pharmaceutically acceptable acid addition salt.

8. A process for obtaining the R-enantiomer of α-[[(1,1-dimethylethyl)-amino]methyl]-4-hydroxy-1,3-benzenedimethanol or a pharmaceutically acceptable salt thereof, which comprises
a) crystallizing the S-enantiomer of α-[[(1,1-dimethylethyl)amino]methyl]-4-hydroxy-1,3-benzenedimethanol as its salt with a chiral acid from a solution or slurry of a mixture of said R- and S-enantiomers in at least one solvent, wherein the chiral acid is S-Ibuprofen, and
b) crystallizing the R-enantiomer of α-[[(1,1-dimethylethyl)amino]methyl]-4-hydroxy-1,3-benzenedimethanol or a salt thereof from the mother liquor obtained in step a).

9. The process according to claim 8, further comprising addition of mixture of the enantiomers of α-[[(1,1-dimethylethyl)amino]methyl]-4-hydroxy-1,3-benzenedimethanol to the mother liquor obtained in step b) and repeating steps a) and b).

10. The process according to any of claims 8 or 9, where in step b) the R-enantiomer of α-[[(1,1-dimethylethyl)amino]methyl]-4-hydroxy-1,3-benzenedimethanol is crystallized as its sulfate, hydrogen sulfate, hydrochloride, its salt with pivalic acid or with R-ibuprofen.

11. The process according to any of claims 8 to 10, wherein the crystallization in step a) is effected from a solution or slurry, wherein the at least one solvent forming the solution or slurry comprises at least one protic solvent, selected from water and C₁-C₄-alkanols.

12. The process according to claim 11, wherein the at least one solvent is a C₁-C₄-alkanol or a mixture of C₁-C₄-alkanols.

13. The process according to any of the preceding claims, wherein α-[[(1,1-dimethylethyl)amino]methyl]-4-hydroxy-1,3-benzenedimethanol is present in the solution or slurry before crystallization as its racemate.

14. The acid addition salt of R-enantiomer of α-[[(1,1-dimethylethyl)amino]methyl]-4-hydroxy-1,3-benzenedimethanol with R-Ibuprofen, wherein the enantiomeric excess with regard to the R-enantiomer is at least 70%.

15. The acid addition salt of S-enantiomer of α-[[(1,1-dimethylethyl)amino]methyl]-4-hydroxy-1 ,3-benzenedimethanol with S-Ibuprofen, wherein the enantiomeric excess with regard to the R-enantiomer is at least 70%.
